(19) Europäisches Patentamt
European Patent Office
Office européen des brevets

(11) Numéro de publication: **0 196 943 B1**

(12) **FASCICULE DE BREVET EUROPEEN**

(45) Date de publication du fascicule du brevet: **24.01.90**

(51) Int. Cl.⁴: **C07D 401/12**, C07D 413/12, C07D 417/12, A61K 31/47

(21) Numéro de dépôt: **86400445.2**

(22) Date de dépôt: **04.03.86**

(54) **Nouveaux dérivés 8-thiotétrahydroquinoléines et leurs sels.**

(30) Priorité: **04.03.85 GB 8505492**

(43) Date de publication de la demande: **08.10.86 Bulletin 86/41**

(45) Mention de la délivrance du brevet: **24.01.90 Bulletin 90/4**

(84) Etats contractants désignés: **AT BE CH DE FR IT LI LU NL SE**

(56) Documents cités:
**EP-A- 0 171 372**
**CH-A- 204 731**
**GB-A- 847 701**

(73) Titulaire: **ROUSSEL-UCLAF, 35, boulevard des Invalides, F-75007 Paris(FR)**

(72) Inventeur: **Clements-Jewery, Stephen, 14 Church Walk, Ashton Keynes Wiltshire(GB)**
Inventeur: **Kennewell, Peter David, 10 St. Helen's View Okus, Swindon Wiltshire(GB)**
Inventeur: **Westwood, Robert, Martagon 6 Rimes Close, Kingston Bagpuize Oxon(GB)**

(74) Mandataire: **Vieillefosse, Jean-Claude et al, Département des Brevets ROUSSEL UCLAF B.P no 9, F-93230 Romainville(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 196 943 B1

ACTORUM AG

## Description

La présente invention concerne de nouvelles 8-thiotétrahydroquinoléines substituées et leurs sels, ainsi que le procédé de préparation et l'application à titre de médicament de ces nouveaux produits.

La demande de brevet Européen 0 187 977 non publiée à la date de dépôt de la présente demande décrit des produits présentant une structure chimique assez proche de celle des produits de la présente demande mais des propriétés pharmacologiques assez sensiblement différentes.

L'invention a pour objet de nouvelle 8-thiotétrahydroquinoléines substituées répondant à la formule (I):

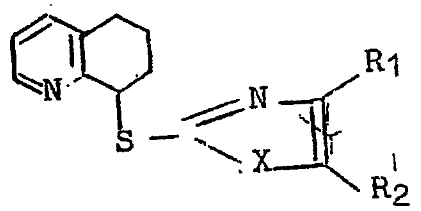

(I)

dans laquelle X représente un atome d'oxygène, de soufre, un groupement –NH–, N-alk dans lequel alk représente un radical alcoyle renfermant de 1 à 6 atomes de carbone, ou un groupement

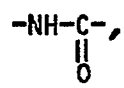

$R_1$ et $R_2$, identiques ou différents, représentent un atome d'hydrogène, un radical phényle ou naphtyle, ou forment ensemble avec les atomes de carbone auxquels ils sont liés, un groupement phényle éventuellement substitué, soit par un radical alcoyle comportant de 1 à 6 atomes de carbone, soit par un radical alcoxy comportant de 1 à 6 atomes de carbone, soit par un radical alcoythio comportant de 1 à 6 atomes de carbone, soit par un ou plusieurs atomes d'halogène, soit par un groupement $-NO_2$, soit par un radical trifluoro-méthyle, soit par un groupement

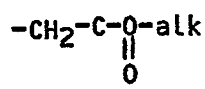

dans lequel alk représente un radical alcoyle renfermant de 1 à 6 atomes de carbone, ainsi que leurs sels d'addition avec les acides minéraux ou organiques.

Dans la formule générale (I) et dans ce qui suit:

– le terme alcoyle comportant de 1 à 6 atomes de carbone désigne, par exemple, un radical méthyle, éthyle, propyle, isopropyle, butyle, linéaire ou ramifié, pentyle linéaire ou ramifie, hexyle linéaire ou ramifié;
– le terme alcoxy comportant de 1 à 6 atomes de carbone désigne, par exemple, un radical méthoxy, éthoxy, propoxy, isopropoxy, butoxy, linéaire ou ramifié, pentyloxy linéaire ou ramifié, hexyloxy linéaire ou ramifié;
– le terme alcoythio comportant de 1 à 6 atomes de carbone désigne, par exemple, un radical méthylthio, éthylthio, propylthio, isopropylthio, butylthio linéaire ou ramifié, pentylthio linéaire ou ramifié, hexylthio linéaire ou ramifié;
– le terme halogène représente notamment le chlore ou le brome.

Les sels d'addition avec les acides minéraux ou organiques peuvent être, par exemple, les sels formés avec les acides chlorhydrique, bromhydrique, iodhydrique, nitrique, sulfurique, phosphorique, propionique, formique, benzoïque, maléique, fumarique, succinique, tartrique, citrique, oxalique, glyoxylique, aspartique, alcane sulfoniques tels que l'acide méthanesulfonique et arylsulfoniques, tels que l'acide benzènesulfonique.

Parmi les produits, objet de l'invention, on peut citer notamment les dérivés répondant à la formule (I) ci-dessus, ainsi que leurs sels, caractérisés en ce que dans ladite formule (I), $R_1$ et $R_2$ représentent ensemble avec les atomes de carbone auxquels ils sont liés, un groupement phényle éventuellement substitué soit par un radical alcoyle comportant de 1 à 4 atomes de carbone, soit par un radical alcoxy comportant de 1 à 4 atomes de carbone, soit par un radical alcoylthio comportant de 1 à 4 atomes de carbone, soit par un ou plusieurs atomes d'halogène, soit par un groupement $-NO_2$, soit par un radical trifluorométhyle, soit par un groupement

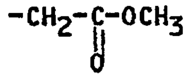 ou 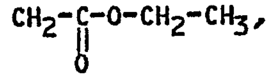

et X a la signification déjà indiquée.

Parmi les produits, objet de l'invention, on peut citer également les dérivés répondant à la formule (I) ci-dessus, ainsi que leurs sels, caractérisé en ce que dans ladite formule (I) X représente un radical –NH et $R_1$ et $R_2$ ont les significations déjà indiqués.

Parmi les produits objet de l'invention, on retient aussi les dérivées répondant à la formule I ci-dessus ainsi que leurs sels, caractérisés en ce que dans ladite formule (I) X représente du soufre et $R_1$ et $R_2$ ont les significations déjà indiquées.

Parmi les produits, objet de l'invention, on peut citer tout particulièrement les dérivés de formule (I), dont les noms suivent :

- la 8-(2-benzothiazolylthio)-5,6,7,8-tétrahydroquinoléine,
- la 8-(6-chloro-1H-benzimidazol-2-ylthio)-5,6,7,8-tétrahydroquinoléine,
- la 8-/(5-chloro-2-benzothiazolyl)thio/-5,6,7,8-tétrahydroquinoléine,
- la 8-/(6-chloro-2-benzothiazolyl)thio/-5,6,7,8-tétrahydroquinoléine,
- la 8-/(6-éthoxy-2-benzothiazolyl)thio/-5,6,7,8-tétrahydroquinoléine,
- la 8-/(6-éthylthio-2-benzothiazolyl)thio/-5,6,7,8-tétrahydroquinoléine,
- la 8-/(6-nitro-2-benzothiazolyl)thio/-5,6,7,8-tétrahydroquinoléine,
- la 8-(4,5-diphényl-1H-imidazol-2-ylthio)-5,6,7,8-tétrahydroquinoléine,

et leurs sels.

L'invention a également pour objet un procédé de préparation des nouvelles 8-thiotétrahydroquinoléines substituées telles que définies par la formule (I) ci-dessus, ainsi que de leurs sels, caractérisé en ce que l'on fait réagir un composé de formule (II) :

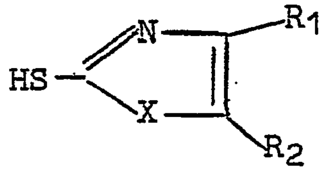

(II)

dans laquelle X, $R_1$, $R_2$ ont les significations déjà indiquées, avec un composé de formule (III) :

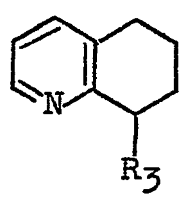

(III)

dans laquelle $R_3$ représente un atome d'halogène tel que le chlore, le brome ou l'iode, pour obtenir le composé de formule (I) désiré :

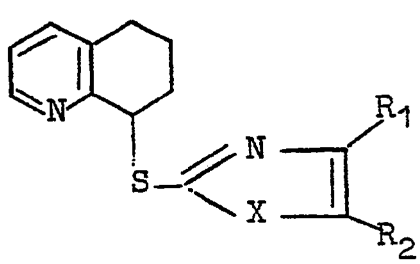

(I)

dans laquelle X, $R_1$, $R_2$ ont les significations déjà indiquées et, si désiré, salifie le produit de formule (I) ainsi obtenu.

Dans des conditions préférentielles de mise en oeuvre de l'invention, le procédé de préparation ci-dessus décrit est caractérisé en ce que :

a/. Le composé de formule (II) est utilisé sous forme de sel alcalin obtenu de préférence par action d'un hydrure de métal alcalin tel que l'hydrure de sodium sur le produit de formule (II).

b/. La réaction des composés de formules (II) et (III) a lieu au sein d'un solvant organique tel que l'éther éthylique ou le tétrahydrofuranne.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques ; ils sont doués notamment de remarquables propriétés inhibitrices de la 5-lipoxygénase et de la liaison de la leukotriène $D_4$ à ses récepteurs. En tant que tels, ils peuvent être utilisés pour leurs propriétés anti-allergiques.

Ces propriétés justifient l'utilisation des nouvelles 8-thiotétrahydroquinoléines substituées de formule (I), ainsi que de leurs sels pharmaceutiquement acceptables, à titre de médicaments.

La présente invention a ainsi également pour objet l'application à titre de médicaments des nouvelles 8-thiotétrahydroquinoléines substituées telles que définies par la formule générale (I) ainsi que de leurs sels d'addition avec les acides pharmaceutiquement acceptables.

Parmi les médicaments, objet de l'invention, on retient notamment les médicaments caractérisés en ce qu'ils sont constitués par les nouvelles 8-thiotétrahydroquinoléines substituées répondant à la formule générale (I) dans laquelle $R_1$ et $R_2$ représentent ensemble avec les atomes de carbone auxquels ils sont liés, un groupement phényle éventuellement substitué soit par un radical alcoyle comportant de 1 à 4 atomes de carbone, soit par un radical alcoxy comportant de 1 à 4 atomes de carbone, soit par un radical alcoythio comportant de 1 à 4 atomes de carbone, soit par un plusieurs atomes d'halogène, soit par un groupement $-NO_2$, soit par un radical trifluorométhyle, soit par un groupement

$$-CH_2-\underset{\underset{O}{\|}}{C}-OCH_3 \text{ ou } -CH_2-\underset{\underset{O}{\|}}{C}-O-CH_2-CH_3$$

et X a la signification déjà indiquée, ainsi que leurs sels d'addition avec les acides pharmaceutiquement acceptables.

Parmi les médicaments, objet de l'invention, on retient plus particulièrement ceux répondant à la formule générale (I) dans laquelle X représente un radical -NH et $R_1$ et $R_2$ ont les significations déjà indiquées ainsi que leurs sels d'addition avec les acides pharmaceutiquement acceptables.

Parmi les médicaments de l'invention, on retient tout particulièrement ceux répondant à la formule (I) dans laquelle X représente du soufre et $R_1$ et $R_2$ ont les significations déjà indiquées ainsi que leurs sels d'addition avec les acides pharmaceutiquement acceptables.

Parmi les médicaments préférés de l'invention, on retient tout particulièrement :

- la 8-(2-benzothiazolyl thio)-5,6,7,8-tétrahydroquinoléine,
- la 8-(6-chloro-1H-benzimidazol-2-yl thio)-5,6,7,8-tétrahydroquinoléine,
- la 8-/(5-chloro-2-benzothiazolyl)thio/-5,6,7,8-tétrahydroquinoléine,
- la 8-/(6-chloro-2-benzothiazolyl)thio/-5,6,7,8-tétrahydroquinoléine,
- la 8-/(6-éthoxy-2-benzothiazolyl)thio/-5,6,7,8-tétrahydroquinoléine,
- la 8-/(6-éthylthio-2-benzothiazolyl)thio/-5,6,7,8-tétrahydroquinoléine,
- la 8-/(6-nitro-2-benzothiazolyl)thio/-5,6,7,8-tétrahydroquinoléine,
- la 8-(4,5-diphényl-1H-imidazol-2-ylthio)-5,6,7,8-tétrahydroquinoléine,

ainsi que leurs sels pharmaceutiquement acceptables.

Ces médicaments trouvent leur emploi dans le traitement des états asthmatiques allergiques et des bronchites d'origine allergique.

La dose usuelle, variable selon le produit utilisé, le sujet traité, et l'affection en cause, peut-être par exemple de 0,1 mg à 200 mg par jour par voie orale.

L'invention a également pour objet les compositions pharmaceutiques, caractérisées en ce qu'elles renferment à titre de principe actif, l'un au moins des médicaments tels que définis précédemment.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émusifiants, les conservateurs.

Les produits de formule (II) et de formule (III) sont décrits dans la littérature.

Les exemples suivants illustrent l'invention, sans toutefois la limiter.

**Exemple 1 :** 8-(2-benzothiazolylthio)-5,6,7,8-tétrahydroquinoléine.

On ajoute sous atmosphère d'azote 2 g d'hydrure de sodium à 80% à 9,97 g de 2-mercaptobenzothiazole dans 250 cm3 de tétrahydrofuranne. On agite la suspension à température ambiante, puis ajoute goutte à goutte 10 g de 8-chloro-5,6,7,8-tétrahydroquinoléine et maintient sous agitation pendant 48 heures. On chasse le solvant sous pression réduite, reprend le résidu dans un mélange d'eau et de chlorure de méthylène, lave la phase organique à l'eau, la sèche et la concentre sous pression réduite. On chromatographie le résidu sur silice (éluant : chlorure de méthylène-éther éthylique 1-0 changeant progressivement jusqu'à 0-1). Après cristallisation dans le mélange chlorure de méthylène-éther de pétrole (Eb = 40°-60°C), on obtient 10,9 g de produit attendu. F = 72°-73°C.

**Spectre RMN** ($CDCl_3$)

$\gamma_H$($CDCl_3$) : 1,95 (1H, mult.), 2,15 (1H, mult.), 2,48 (2H, mult.), 2,84 (2H, mult.), 5,50 (1H, triplet, J = 5Hz), 7,14 (1H, mult.), 7,38 (3H, mult.), 7,76 (1H, mult.), 7,91 (1H, mult.), et 8,49 (1H, mult.).

Analyse: $C_{16}H_{14}N_2S_2$

| | C% | H% | N% | S% |
|---|---|---|---|---|
| Calculé: | 64,39 | 4,74 | 9,38 | 21,49 |
| Trouvé: | 64,45 | 4,81 | 9,35 | 21,20 |

Exemples 2 à 27 :

En utilisant une méthode analogue à celle décrite à l'exemple 1 mais au départ des produits de formule II correspondants, on a préparé les produits des exemples 2 à 27, le rendement, le point de fusion et les résultats de la microanalyse figurent dans le tableau I ci-après.

**Exemple 2 :** 8-(6-méthoxy-1H-benzimidazol-2-ylthio)-5,6,7,8-tétrahydroquinoléine.
**Exemple 3 :** 8-(1H-benzimidazol-2-ylthio)-5,6,7,8-tétrahydroquinoléine.
**Exemple 4 :** éthyl 2-/(5,6,7,8-tétrahydro-8-quinolinyl)thio/-1H-benzimidazole-5-acétate.
**Exemple 5 :** 8-(6-nitro-1H-benzimidazol-2-ylthio)-5,6,7,8-tétrahydroquinoléine.
**Exemple 6 :** 8-(6-méthyl-1H-benzimidazol-2-ylthio)-5,6,7,8-tétrahydroquinoléine.
**Exemple 7 :** 8-(5,6-dichloro-1H-benzimidazol-2-ylthio)-5,6,7,8,-tétrahydroquinoléine.
**Exemple 8 :** 8-(6-chloro-1H-benzimidazol-2-ylthio)-5,6,7,8-tétrahydroquinoléine.
**Exemple 9 :** 8-(2-benzoxazolylthio)-5,6,7,8-tétrahydroquinoléine.
**Exemple 10 :** méthyl 2-/(5,6,7,8-tétrahydro-8-quinolinyl)thio/benzoxazol-5-acétate.
**Exemple 11 :** 8-/(5-chloro-2-benzothiazolyl)thio/-5,6,7,8-tétrahydroquinoléine.
**Exemple 12 :** 8-/(6-chloro-2-benzothiazolyl)thio/-5,6,7,8-tétrahydroquinoléine.
**Exemple 13 :** 8-/(6-éthoxy-2-benzothiazolyl)thio/-5,6,7,8-tétrahydroquinoléine.
**Exemple 14 :** 2-/(5,6,7,8-tétrahydroquinolin-8-yl)thio/-4(3H)-quinazolinone.
**Exemple 15 :** 8-/(6-méthyl-2-benzothiazolyl)thio/-5,6,7,8-tétrahydroquinoléine.
**Exemple 16 :** 8-/(5-nitro-2-benzothiazolyl)thio/-5,6,7,8-tétrahydroquinoléine.
**Exemple 17 :** 8-/(5-méthyl-2-benzothiazolyl)thio/-5,6,7,8-tétrahydroquinoléine.
**Exemple 18 :** 8-/(5,6-dichloro-2-benzothiazolyl)thio/-5,6,7,8-tétrahydroquinoléine.
**Exemple 19 :** 8-/(6-éthylthio-2-benzothiazolyl)thio/-5,6,7,8-tétrahydroquinoléine.
**Exemple 20 :** 8-/(6-nitro-2-benzothiazolyl)thio/-5,6,7,8-tétrahydroquinoléine.
**Exemple 21 :** 8-/(5-trifluorométhyl-2-benzothiazolyl)thio/-5,6,7,8-tétrahydroquinoléine.
**Exemple 22 :** 8-/(4,6-dichloro-2-benzothiazolyl)thio/-5,6,7,8-tétrahydroquinoléine.
**Exemple 23 :** 8-/(4-chloro-2-benzothiazolyl)thio/-5,6,7,8-tétrahydroquinoléine.
**Exemple 24 :** 8-/(4-méthyl-2-benzothiazolyl)thio/-5,6,7,8-tétrahydroquinoléine.
**Exemple 25 :** 8-(4,5-diphényl-1H-imidazol-2-ylthio)-5,6,7,8-tétrahydroquinoléine.
**Exemple 26 :** 8-(1-méthylimidazol-2-ylthio)-5,6,7,8-tétrahydroquinoléine.
**Exemple 27 :** 8-(2-thiazolylthio)-5,6,7,8-tétrahydroquinoléine.

TABLEAU I

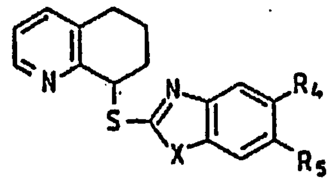

| Exemple | $R_4$ | $R_5$ | X | Rendt (%) | F (°C) | Formule | Analyse Calculé/Trouvé C % | H % | N % | S% | Cl% |
|---|---|---|---|---|---|---|---|---|---|---|---|
| 1 | H | H | S | 61 | 73 | $C_{16}H_{14}N_2S_2$ | 64.39<br>64.45 | 4.74<br>4.81 | 9.38<br>9.35 | 21.49<br>21.20 | |
| 2 | H | OMe | NH | 31 | 159–161 | $C_{17}H_{17}N_3OS.2HCl$<br>$1.5H_2O$ | 49.64<br>49.36 | 5.39<br>5.38 | 10.22<br>10.16 | 7.79<br>7.85 | 17.24<br>17.41 |
| 3 | H | H | NH | 55 | 195 | $C_{16}H_{15}N_3S.2HCl$ | 54.24<br>54.02 | 4.85<br>4.85 | 11.85<br>11.84 | 9.05<br>9.18 | 20.01<br>20.22 |
| 4 | $CH_2CO_2Et$ | H | NH | 29 | 110–111 | $C_{20}H_{21}N_3O_2S.2HCl$ | | | | | - |
| 5 | H | $NO_2$ | NH | 74 | 109–110 | $C_{16}H_{14}N_4O_2S.H_2O$ | 55.80<br>55.74 | 4.68<br>4.71 | 16.27<br>16.29 | 9.31<br>9.31 | -<br>- |
| 6 | H | $CH_3$ | NH | 49 | 178 | $C_{17}H_{17}N_3S.2HCl$ | 55.43<br>55.18 | 5.21<br>5.31 | 11.40<br>11.17 | 8.70<br>8.60 | 19.25<br>19.12 |

TABLEAU I (suite)

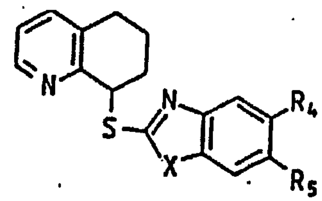

| Exemple | $R_4$ | $R_5$ | X | Rendt (%) | F (°C) | Formule | Analyse Calculé/Trouvé C% | H% | N% | S% | Cl % |
|---|---|---|---|---|---|---|---|---|---|---|---|
| 7 | Cl | Cl | NH | 45 | 105-6 | $C_{16}H_{13}N_3Cl_2S.H_2O$ | 52.18<br>52.26 | 4.11<br>4.17 | 11.41<br>11.45 | 8.74<br>8.74 | 19.25<br>19.27 |
| 8 | H | Cl | NH | 44 | 162-66 | $C_{16}H_{14}N_3ClS.2HCl$ | 49.43<br>49.25 | 4.16<br>4.24 | 10.80<br>10.65 | 8.25<br>8.26 | 27.36<br>27.25 |
| 9 | H | H | O | 48 | 68-71 | $C_{16}H_{14}N_2OS$ | 68.05<br>67.50 | 5.01<br>5.03 | 9.92<br>9.89 | 11.35<br>11.39 | -<br>- |
| 10 | $CH_2CO_2Me$ | H | O | 25 | 87-88 | $C_{19}H_{18}N_2O_3S$ | 64.38<br>64.28 | 5.13<br>5.15 | 7.90<br>7.93 | 9.05<br>9.17 | -<br>- |
| 11 | Cl | H | S | 61 | 155-156 | $C_{16}H_{13}N_2ClS_2.HCl$ | 52.03<br>51.80 | 3.83<br>3.95 | 7.58<br>7.52 | 17.36<br>17.00 | 19.20<br>19.67 |
| 12 | H | Cl | S | 82 | 169 | $C_{16}H_{13}N_2ClS_2.HCl$ | 52.03<br>52.02 | 3.83<br>3.88 | 7.58<br>7.64 | 17.36<br>17.22 | 19.20<br>19.45 |

TABLEAU I (suite)

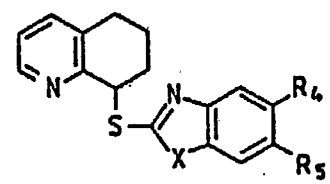

| Exemple | $R_4$ | $R_5$ | X | Rendt (%) | F (°C) | Formule | Analyse Calculé/Trouvé C% | H% | N% | S% | Cl% |
|---|---|---|---|---|---|---|---|---|---|---|---|
| 13 | H | OEt | S | 71 | 156-8 | $C_{18}H_{18}N_2OS_2$.HCl | 57.05<br>57.08 | 5.06<br>5.10 | 7.39<br>7.38 | 16.92<br>16.69 | 9.36<br>9.69 |
| 14 | H | H | NHCO | 45 | 192-3 | $C_{17}H_{15}N_3OS$ | 65.99<br>65.68 | 4.90<br>4.99 | 13.58<br>13.40 | 10.36<br>10.27 | |
| 15 | H | Me | S | 56 | 162-4* | $C_{17}H_{16}N_2S_2$.HCl | 58.52<br>58.23 | 4.92<br>5.05 | 8.02<br>7.82 | | |
| 16 | $NO_2$ | H | S | 43 | 130-1 | $C_{16}H_{13}N_3O_2S_2$ | 55.96<br>55.58 | 3.82<br>3.85 | 12.23<br>12.13 | 18.67<br>18.32 | |
| 17 | Me | H | S | 18 | 155-7* | $C_{17}H_{16}N_2S_2$.HCl | 58.52<br>58.31 | 4.92<br>4.94 | 8.02<br>8.01 | | 10.16<br>10.33 |
| 18 | Cl | Cl | S | 30 | 165-7* | $C_{16}H_{12}N_2S_2Cl_2$<br>.HCl | 47.59<br>47.55 | 3.25<br>3.29 | 6.93<br>7.00 | 15.88 | 26.34<br>26.02 |
| 19 | H | SEt | S | 51 | 138-40* | $C_{18}H_{18}N_2S_3$.HCl | 54.73<br>54.52 | 4.86<br>4.89 | 7.09<br>7.07 | 24.35<br>23.80 | 8.98<br>9.20 |

* = décomposition

TABLEAU I (suite)

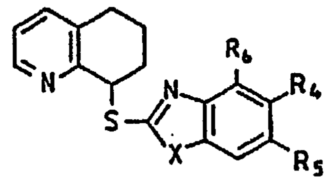

| Exemple | $R_4$ | $R_5$ | $R_6$ | X | Rendt (%) | F (°C) | Formule | Analyse Calculé/Trouvé C% | H% | N% | S% | Cl% |
|---|---|---|---|---|---|---|---|---|---|---|---|---|
| 20 | H | $NO_2$ | H | S | 30 | 161-2 | $C_{16}H_{13}N_3O_2S_2$ .HCl | 50.59<br>50.58 | 3.71<br>3.78 | 11.06<br>11.05 | | 9.33<br>9.27 |
| 21 | $CF_3$ | H | H | S | 18 | 119-29 | $C_{17}H_{13}F_3N_2S_2$ .HCl | 50.68<br>50.62 | 3.50<br>3.55 | 6.95<br>6.89 | | 8.80<br>8.70 |
| 22 | H | Cl | Cl | S | 15 | 158-61* | $C_{16}H_{12}N_2S_2Cl_2$ .HCl | 47.59<br>47.49 | 3.25<br>3.28 | 6.93<br>6.93 | 15.88<br>15.64 | 26.34<br>26.27 |
| 23 | H | H | Cl | S | 49 | 154-8 | $C_{16}H_{13}N_2S_2Cl$ .HCl | 52.03<br>52.06 | 3.82<br>3.91 | 7.58<br>7.55 | | 19.20<br>19.29 |
| 24 | H | H | Me | S | 46 | 131-8 | $C_{17}H_{16}N_2S_2$.HCl | 58.52<br>58.27 | 4.91<br>4.96 | 8.03<br>8.00 | | 10.16<br>10.38 |

* = décomposition

TABLEAU I (Suite)

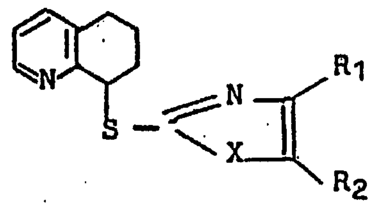

| Example | $R_1$ | $R_2$ | X | Rendt (%) | F (°C) | Formule | Analyse Calculé/Trouvé C% | H% | N% | S% | Cl% |
|---|---|---|---|---|---|---|---|---|---|---|---|
| 25 | Ph | Ph | NH | 51 | 181-2 | $C_{24}H_{21}N_3S.2HCl$ | 63.15<br>62.90 | 5.09<br>5.16 | 9.20<br>9.06 | 7.02<br>6.96 | 15.53<br>15.60 |
| 26 | H | H | NMe | 42 | 178-9 | $C_{13}H_{15}N_3S.2HCl$<br>$.H_2O$ | 46.43<br>46.20 | 5.69<br>5.64 | 12.50<br>12.51 | 9.53<br>9.61 | 21.09<br>21.01 |
| 27 | H | H | S | 41 | 118-20 | $C_{12}H_{12}N_2S_2.2HCl$<br>½ EtOH | 45.20<br>44.57 | 4.80<br>4.88 | 8.32<br>8.21 | 19.05<br>19.65 | 21.06<br>20.67 |

Exemple 28:

On a préparé des comprimés répondant à la formulation suivante:
- Produit de l'exemple 13 .................................................. 20 mg
- Excipient q.s. pour un comprimé terminé à .................... 100 mg.
(Détail de l'excipient: lactose, amidon, talc, stéarate de magnésium).

Exemple 29:

On a préparé des comprimés répondant à la formulation suivante:
- Produit de l'exemple 11 .................................................. 20 mg
- Excipient q.s. pour un comprimé terminé à .................... 100 mg.
(Détail de l'excipient: lactose, amidon, talc, stéarate de magnésium).

Exemple 30:

On a préparé un aérosol délivrant par dose:
- Produit de l'exemple 1 ............................................... 2 mg
- Emulsifiant ............................................................ 0,15 mg
- Propulseur ............................................................. 50 mg.

ETUDE BIOCHIMIQUE

5-Lipox

L'inhibition de l'ionophore $Ca^{++}$ (A 23187) provoque la libération de produits 5-lipoxygénase (Leukotriene $B_4$ et 5-HETE) de l'acide /14c/ arachidonique des neutrophiles péritonéales pré-marqués du rat.

On a utilisé la méthode de Ahnfelt-Ronne, I. et Arrigoni-Martelli, E. Biochemical Pharmacology, Vol. 31, N¤ 16, p. 2619–2624 (1982) modifiée. Les valeurs indiquées sont des concentrations micromolaires du composé testé provoquant 50% d'inhibition par rapport à la réponse du témoin déterminés graphiquement d'après les courbes de réponse par dose.

Récepteur $LTD_4$

Etude de l'inhibition de la liaison spécifique du /$^3$H/ $LTD_4$ sur une préparation de membrane de tissu de poumon de cobaye. L'évaluation a été effectuée par une méthode modifiée de Bruns, R.F. Thomsen, W.J. et Pugsley, T.A. Life Sciences, Vol. 33, p. 645–653, (1983). Les valeurs indiquées sont des concentrations micromolaires du composé testé provoquant 50% d'inhibition de la liaison spécifique déterminée graphiquement d'après les courbes de réponse par dose.

Les résultats de ces tests sont donnés dans le tableau II:

Tableau II

| Exemple | 5-LIPOX | récepteur $LTD_4$ |
|---|---|---|
| 1 | 1,8 | 10 |
| 2 | 58,0 | |
| 3 | 35,1 | > 100 |
| 4 | 20,0 | |
| 5 | 6,6 | > 100 |
| 6 | 5,0 | 74 |
| 7 | 4,2 | |
| 8 | 1,9 | 6,3 |
| 9 | 16,9 | > 100 |
| 10 | 14,1 | 12 |
| 11 | 1,5 | 100 |
| 12 | 2,6 | |
| 13 | 1,2 | |
| 14 | 3,0 | > 100 pot. |
| 15 | > 100 | > 100 |
| 16 | > 100 | > 100 |
| 17 | 25,1 | > 100 |

**Revendications pour les Etats contractants: CH, DE, FR, IT, LI, NL, SE**

1. Nouvelles 8-thiotétrahydroquinoléines substituées répondant à la formule (I):

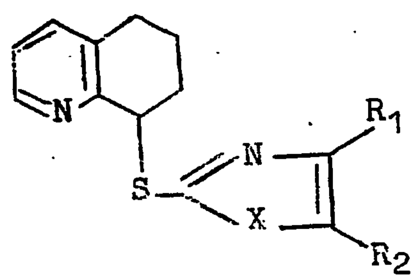

(I)

dans laquelle X représente un atome d'oxygène, de soufre, un groupement –NH–, N-alk dans lequel alk représente un radical alcoyle renfermant de 1 à 6 atomes de carbone, ou un groupement

$$-NH-\underset{\underset{O}{\|}}{C}-,$$

$R_1$ et $R_2$, identiques ou différents, représentent un atome d'hydrogène, un radical phényle ou naphtyle, ou forment ensemble avec les atomes de carbone auxquels ils sont liés, un groupement phényle éventuellement substitué, soit par un radical alcoyle comportant de 1 à 6 atomes de carbone, soit par un radical alcoxy comportant de 1 à 6 atomes de carbone, soit par un radical alcoythio comportant de 1 à 6 atomes de carbone, soit par un ou plusieurs atomes d'halogène, soit par un groupement $-NO_2$, soit par un radical trifluoro-méthyle, soit par un groupement

$$-CH_2-\underset{\underset{O}{\|}}{C}-O-alk$$

dans lequel alk représente un radical alcoyle renfermant de 1 à 6 atomes de carbone, étant entendu que X ne peut représenter un groupement –NH– lorsque $R_1$ et $R_2$ forment ensemble avec les atomes de carbone auxquels ils sont liés un groupement phényle éventuellement substitué, ainsi que leurs sels d'addition avec les acides minéraux ou organiques.

2. Nouvelles 8-thiotétrahydroquinoléines substituées telles que définies à la revendication 1, et leurs sels, caractérisée en ce que dans ladite formule (I), $R_1$ et $R_2$ représentent ensemble avec les atomes de carbone auxquels ils sont liés, un groupement phényle éventuellement substitué soit par un radical alcoyle comportant de 1 à 4 atomes de carbone, soit par un radical alcoxy comportant de 1 à 4 atomes de carbone, soit par un radical alcoylthio comportant de 1 à 4 atomes de carbone, soit par un plusieurs atomes d'halogène, soit par un groupement $-NO_2$, soit par un radical trifluorométhyle, soit par un groupement

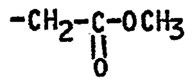

ou

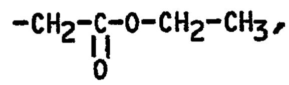

et X a la signification déjà indiquée.

3. Nouvelles 8-thiotétrahydroquinoléines substituées telles que définies à la revendication 1 ou 2, et leurs sels, caractérisées en ce que ce que dans ladite formule (I), X représente un atome de soufre ou un radical –NH et $R_1$ et $R_2$ ont les significations déjà indiquées.

4. L'un quelconque des dérivés répondant à la formule (I) de la revendication 1, dont les noms suivent:
– la 8-(2-benzothiazolylthio)-5,6,7,8-tétrahydroquinoléine,
– la 8-[(5-chloro-2-benzothiazolyl)thio]-5,6,7,8-tétrahydroquinoléine,
– la 8-[(6-chloro-2-benzothiazolyl)thio]-5,6,7,8-tétrahydroquinoléine,
– la 8-[(6-éthoxy-2-benzothiazolyl)thio]-5,6,7,8-tétrahydroquinoléine,
– la 8-[(6-éthylthio-2-benzothiazolyl)thio]-5,6,7,8-tétrahydroquinoléine,
– la 8-[(6-nitro-2-benzothiazolyl)thio]-5,6,7,8-tétrahydroquinoléine,
– la 8-(4,5-diphényl-1H-imidazol-2-ylthio)-5,6,7,8-tétrahydroquinoléine,
et leurs sels.

5. Procédé de préparation des nouvelles 8-thiotétrahydroquinoléines substituées telles que définies par la formule (I) de la revendication 1, ainsi que de leurs sels, caractérisé en ce que l'on fait réagir un composé de formule (II):

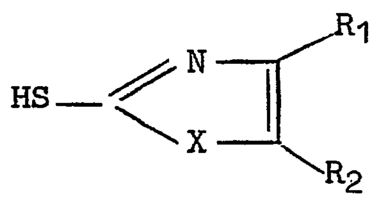

(II)

dans laquelle X, $R_1$, $R_2$ ont les significations déjà indiquées, avec un composé de formule (III):

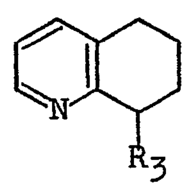

(III)

dans laquelle $R_3$ représente un atome d'halogène pour obtenir le composé de formule (I) désiré:

(I)

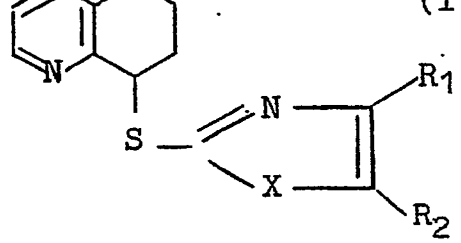

dans laquelle X, $R_1$, $R_2$ ont les significations déjà indiquées et, si désiré, salifie le produit de formule (I) ainsi obtenu.

6. Procédé de préparation selon la revendication 5, caractérisé en ce que:
– l'atome d'halogène que représente $R_3$ est un atome de chlore, de brome ou d'iode,
– le composé de formule (II) utilisé est sous forme de sel alcalin obtenu de préférence par action d'un hydrure de métal alcalin sur le composé de formule (II),
– la réaction des composés de formules (II) et (III) a lieu au sein d'un solvant organique.

7. Médicaments, caractérisé en ce qu'ils sont constitués par les nouvelles 8-thiotétrahydroquinoléines substituées telles que définies par la formule (I) de la revendication 1, ainsi que leurs sels pharmaceutiquement acceptables.

8. Médicaments, caractérisé en ce qu'ils sont constitués par les nouvelles 8-thiotétrahydroquinoléines substituées telles que définies à l'une quelconque des revendications 2 ou 3, ainsi que leurs sels pharmaceutiquement acceptables.

9. Médicaments, caractérisé en ce qu'ils sont constitués par les nouvelles 8-thiotétrahydroquinoléines substituées telles que définies à la revendication 4, ainsi que leurs sels pharmaceutiquement acceptables.

10. Compositions pharmaceutiques, caractérisées en ce qu'elles renferment à titre de principe actif, l'un au moins des médicaments tels que définis à l'une quelconque des revendications 7 à 9.

**Revendications pour les Etats contractants: AT, BE, LU**

1. Nouvelles 8-thiotétrahydroquinoléines substituées répondant à la formule (I):

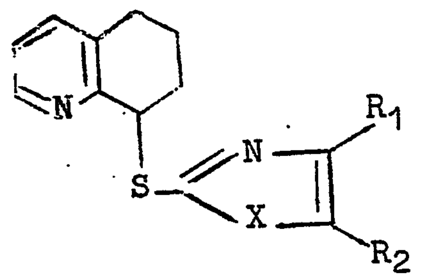

(I)

dans laquelle X représente un atome d'oxygène, de soufre, un groupement –NH–, N-alk dans lequel alk représente un radical alcoyle renfermant de 1 à 6 atomes de carbone, ou un groupement

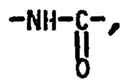

$R_1$ et $R_2$, identiques ou différents, représentent un atome d'hydrogène, un radical phényle ou naphtyle, ou forment ensemble avec les atomes de carbone auxquels ils sont liés, un groupement phényle éventuellement substitué, soit par un radical alcoyle comportant de 1 à 6 atomes de carbone, soit par un radical alcoxy comportant de 1 à 6 atomes de carbone, soit par un radical alcoythio comportant de 1 à 6 atomes de carbone, soit par un ou plusieurs atomes d'halogène, soit par un groupement $-NO_2$, soit par un radical trifluoro-méthyle, soit par un groupement

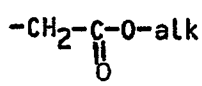

dans lequel alk représente un radical alcoyle renfermant de 1 à 6 atomes de carbone, ainsi que leurs sels d'addition avec les acides minéraux ou organiques.

2. Nouvelles 8-thiotétrahydroquinoléines substituées telles que définies à la revendication 1, et leurs sels, caractérisées en ce que dans ladite formule (I), $R_1$ et $R_2$ représentent ensemble avec les atomes de carbone auxquels ils sont liés, un groupement phényle éventuellement substitué soit par un radical alcoyle comportant de 1 à 4 atomes de carbone, soit par un radical alcoxy comportant de 1 à 4 atomes de carbone, soit par un radical alcoylthio comportant de 1 à 4 atomes de carbone, soit par un plusieurs atomes d'halogène, soit par un groupement $-NO_2$, soit par un radical trifluorométhyle, soit par un groupement

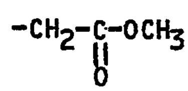

ou

$$-CH_2-\underset{\underset{O}{\|}}{C}-O-CH_2-CH_3,$$

et X a la signification déjà indiquée.

3. Nouvelles 8-thiotétrahydroquinoléines substituées telles que définies à la revendication 1 ou 2, et leurs sels, caractérisées en ce que ce que dans ladite formule (I), X représente un atome de soufre ou un radical –NH et $R_1$ et $R_2$ ont les significations déjà indiquées.

4. L'un quelconque des dérivés répondant à la formule (I) de la revendication 1, dont les noms suivent:
– la 8-(2-benzothiazolylthio)-5,6,7,8-tétrahydroquinoléine,
– la 8-(6-chloro-1H-benzimidazol-2-ylthio)-5,6,7,8-tétrahydroquinoléine,
– la 8-[(5-chloro-2-benzothiazolyl)thio]-5,6,7,8-tétrahydroquinoléine,
– la 8-[(6-chloro-2-benzothiazolyl)thio]-5,6,7,8-tétrahydroquinoléine,
– la 8-[(6-éthoxy-2-benzothiazolyl)thio]-5,6,7,8-tétrahydroquinoléine,
– la 8-[(6-éthylthio-2-benzothiazolyl)thio]-5,6,7,8-tétrahydroquinoléine,
– la 8-[(6-nitro-2-benzothiazolyl)thio]-5,6,7,8-tétrahydroquinoléine,
et leurs sels.

5. Procédé de préparation des nouvelles 8-thiotétrahydroquinoléines substituées telles que définies par la formule (I) de la revendication 1, ainsi que de leurs sels, caractérisé en ce que l'on fait réagir un composé de formule (II):

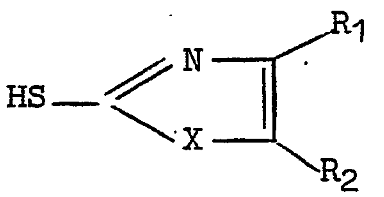

(II)

dans laquelle X, $R_1$, $R_2$ ont les significations déjà indiquées, avec un composé de formule (III):

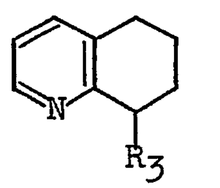

(III)

dans laquelle $R_3$ représente un atome d'halogène pour obtenir le composé de formule (I) désiré:

(I)

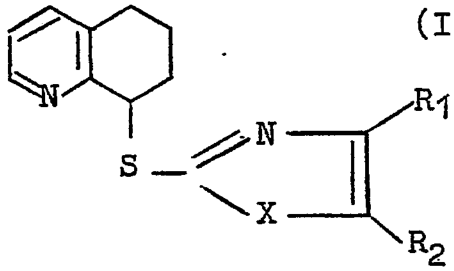

dans laquelle X, $R_1$, $R_2$ ont les significations déjà indiquées et, si désiré, salifie le produit de formule (I) ainsi obtenu.

6. Procédé de préparation selon la revendication 5, caractérisé en ce que:
– l'atome d'halogène que représente $R_3$ est un atome de chlore, de brome ou d'iode,
– le composé de formule (II) utilisé est sous forme de sel alcalin obtenu de préférence par action d'un hydrure de métal alcalin sur le composé de formule (II),
– la réaction des composés de formules (II) et (III) a lieu au sein d'un solvant organique.

7. Médicaments, caractérisés en ce qu'ils sont constitués par les nouvelles 8-thiotétrahydroquinoléines substituées telles que définies par la formule (I) de la revendication 1, ainsi que leurs sels pharmaceutiquement acceptables.

8. Médicaments, caractérisés en ce qu'ils sont constitués par les nouvelles 8-thiotétrahydroquinoléines substituées telles que définies à l'une quelconque des revendications 2 ou 3, ainsi que leurs sels pharmaceutiquement acceptables.

9. Médicaments, caractérisés en ce qu'ils sont constitués par les nouvelles 8-thiotétrahydroquinoléines substituées telles que définies à la revendication 4, ainsi que leurs sels pharmaceutiquement acceptables.

10. Compositions pharmaceutiques, caractérisées en ce qu'elles renferment à titre de principe actif, l'un au moins des médicaments tels que définis à l'une quelconque des revendications 7 à 9.

**Claims for the contracting states: CH, DE, FR, IT, LI, NL, SE**

1. New substituted 8-thiotetrahydroquinolines corresponding to the formula (I):

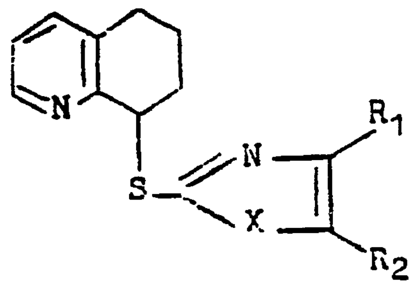

(I)

in which X represents an oxygen or sulphur atom, an –NH–, N-alk group in which alk represents an alkyl radical containing 1 to 6 carbon atoms, or an

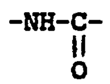

group, $R_1$ and $R_2$, identical or different, represent a hydrogen atom, a phenyl or naphthyl radical, or form, together with the carbon atoms to which they are linked, a phenyl group optionally substituted either by an alkyl radical containing 1 to 6 carbon atoms, or by an alkoxy radical containing 1 to 6 carbon atoms, or by an alkylthio radical containing 1 to 6 carbon atoms, or by one or more halogen atoms, or by an $-NO_2$ group, or by a trifluoromethyl radical, or by a

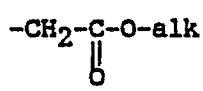

group in which alk represents an alkyl radical containing 1 to 6 atoms, it being understood that X cannot represent an –NH– group when $R_1$ and $R_2$ form, together with the carbon atoms to which they are linked, an optionally substituted phenyl group, as well as their addition salts with mineral or organic acids.

2. New substituted 8-thiotetrahydroquinolines as defined in claim 1, and their salts, characterized in that in the said formula (I), $R_1$ and $R_2$ represent, together with the carbon atoms to which they are linked, a phenyl group optionally substituted either by an alkyl radical containing 1 to 4 carbon atoms, or by an alkoxy radical containing 1 to 4 carbon atoms, or by an alkylthio radical containing 1 to 4 carbon atoms, or by one or more halogen atoms, or by an $-NO_2$ group, or by a trifluoromethyl radical, or by a

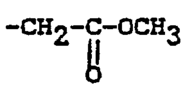 or 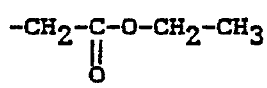

group, and X has the meaning already indicated.

3. New substituted 8-thiotetrahydroquinolines as defined in claim 1 or 2, and their salts, characterized in that in the said formula (I), X represents a sulphur atom or an –NH radical and $R_1$ and $R_2$ have the meanings already indicated.

4. Any one of the derivatives corresponding to the formua (I) of claim 1, of which the names follow:

– 8-(2-benzothiazolylthio)-5,6,7,8-tetrahydroquinoline,
– 8-[(5-chloro-2-benzothiazolyl)thio]-5,6,7,8-tetrahydroquinoline,
– 8-[(6-chloro-2-benzothiazolyl)thio]-5,6,7,8-tetrahydroquinoline,
– 8-[(6-ethoxy-2-benzothiazolyl)thio]-5,6,7,8-tetrahydroquinoline,
– 8-[(6-ethylthio-2-benzothiazolyl)thio]-5,6,7,8-tetrahydroquinoline,
– 8-[6-nitro-2-benzothiazolyl)thio]-5,6,7,8-tetrahydroquinoline,
– 8-(4,5-diphenyl-1H-imidazol-2-yl-thio)-5,6,7,8-tetrahydroquinoline,

and their salts.

5. Process for the preparation of the new substituted 8-thiotetrahydroquinolines as defined by the formula (I) of claim 1, as well as of their salts, characterized in that a compound of formula (II):

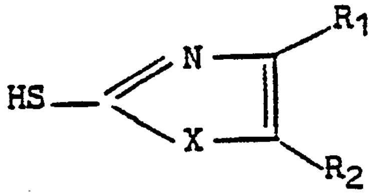

(II)

in which X, $R_1$, $R_2$ have the meanings already indicated, is reacted with a compound of formula (III):

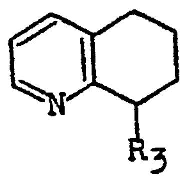

(III)

in which $R_3$ represents a halogen atom, so as to obtain the desired compound of formula (I):

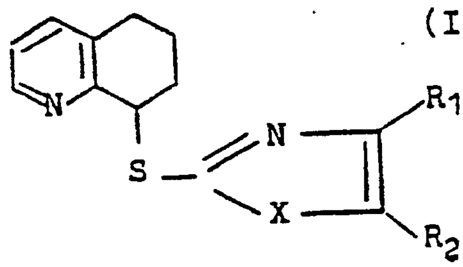

(I)

(I)

in which X, $R_1$, $R_2$ have the meanings already indicated and, if desired, the product of formula (I) thus obtained is salified.

6. Preparation process according to claim 5, characterized in that:

– the halogen atom represented by $R_3$ is a chlorine, bromine or iodine atom,

– the compound of formula (II) used is in the form of an alkaline salt, preferably obtained by the action of an alkali metal hydride on the compound of formula (II),

– the reaction of the compounds of formulae (II) and (III) takes place in an organic solvent.

7. Medicaments, characterized in that they are constituted by the new substituted 8-thiotetrahydroquinolines as defined by formula (I) of claim 1, as well as by their pharmaceutically acceptable salts.

8. Medicaments, characterized in that they are constituted by the new substituted 8-thiotetrahydroquinolines as defined in any one of claims 2 or 3, as well as by their pharmaceutically acceptable salts.

9. Medicaments, characterized in that they are constituted by new substituted 8-thiotetrahydroquinolines as defined in claim 4, as well as by their pharmaceutically acceptable salts.

10. Pharmaceutical compositions, characterized in that they contain as active principle, at least one of the medicaments as defined in any one of claims 7 to 9.

**Claims for the contracting states: AT, BE, LU**

1. New substituted 8-thiotetrahydroquinolines corresponding to the formula (I):

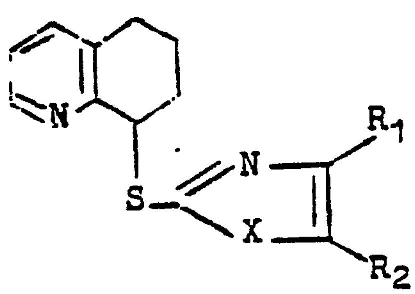

(I)

in which X represents an oxygen or sulphur atom, an –NH–, N-alk group in which alk represents an alkyl radical containing 1 to 6 carbon atoms, or an

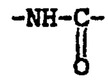

group, $R_1$ and $R_2$, identical or different, represent a hydrogen atom, a phenyl or naphthyl radical, or form, together with the carbon atoms to which they are linked, a phenyl group optionally substituted either by an alkyl radical containing 1 to 6 carbon atoms, or by an alkoxy radical containing 1 to 6 carbon atoms, or by an alkylthio radical containing 1 to 6 carbon atoms, or by one or more halogen atoms, or by an $-NO_2$ group, or by a trifluoromethyl radical, or by a

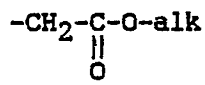

group in which alk represents an alkyl radical containing 1 to 6 carbon atoms, as well as their addition salts with mineral or organic acids.

2. New substituted 8-thiotetrahydroquinolines as defined in claim 1, and their salts, characterized in that in the said formula (I), $R_1$ and $R_2$ represent, together with the carbon atoms to which they are linked, a phenyl group optionally substituted either by an alkyl radical containing 1 to 4 carbon atoms, or by an alkoxy radical containing 1 to 4 carbon atoms, or by an alkylthio radical containing 1 to 4 carbon atoms, or by one or more halogen atoms, or by an $-NO_2$ group, or by a trifluoromethyl radical, or by a

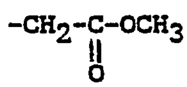 or 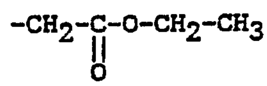

group, and X has the meaning already indicated.

3. New substituted 8-thiotetrahydroquinolines as defined in claim 1 or 2, and their salts, characterized in that in the said formula (I), X represents a sulphur atom or an –NH radical and $R_1$ and $R_2$ have the meanings already indicated.

4. Any one of the derivatives corresponding to the formula (I) of claim 1, of which the names follow:
– 8-(2-benzothiazolylthio)-5,6,7,8-tetrahydroquinoline,
– 8-(6-chloro-1H-benzimidazol-2-yl-thio)-5,6,7,8-tetrahydroquinoline,
– 8-[(5-chloro-2-benzothiazolyl)thio]-5,6,7,8-tetrahydroquinoline,
– 8-[(6-chloro-2-benzothiazolyl)thio]-5,6,7,8-tetrahydroquinoline,
– 8-[(6-ethoxy-2-benzothiazolyl)thio]-5,6,7,8-tetrahydroquinoline,
– 8-[(6-ethylthio-2-benzothiazolyl)thio]-5,6,7,8-tetrahydroquinoline,
– 8-[(6-nitro-2-benzothiazolyl)thio]-5,6,7,8-tetrahydroquinoline,
– 8-(4,5-diphenyl-1H-imidazol-2-yl-thio)-5,6,7,8-tetrahydroquinoline,
and their salts.

5. Process for the preparation of the new substituted 8-thiotetrahydroquinolines as defined by formula (I) of claim 1, as well as of their salts, characterized in that a compound of formula (II):

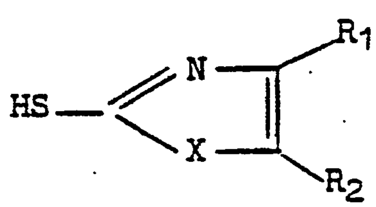

(II)

in which X, $R_1$, $R_2$ have the meanings already indicated, is reacted with a compound of formula (III):

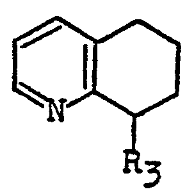

(III)

in which $R_3$ represents a halogen atom, so as to obtain the desired compound of formula (I):

(I)

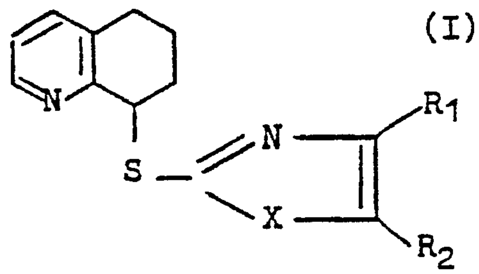

(I)

in which X, $R_1$, $R_2$ have the meanings already indicated and, if desired, the product of formula (I) thus obtained is salified.

6. Preparation process according to claim 5, characterized in that:
- the halogen atom represented by $R_3$ is a chlorine, bromine or iodine atom,
- the compound of formula (II) used is in the form of an alkaline salt, preferably obtained by the action of an alkali metal hydride on the compound of formula (II),
- the reaction of the compounds of formulae (II) and (III) takes place in an organic solvent.

7. Medicaments, characterized in that they are constituted by the new substituted 8-thiotetrahydroquinolines as defined by formula (I) of claim 1, as well as by their pharmaceutically acceptable salts.

8. Medicaments, characterized in that they are constituted by the new substituted 8-thiotetrahydroquinolines as defined in any one of claims 2 or 3, as well as by their pharmaceuticallly acceptable salts.

9. Medicaments, characterized in that they are constituted by new substituted 8-thiotetrahydroquinolines as defined in claim 4, as well as by their pharmaceutically acceptable salts.

10. Pharmaceutical compositions, characterized in that they contain as active principle, at least one of the medicaments as defined in any one of claims 7 to 9.

**Patentansprüche für die Vertragsstaaten: CH, DE, FR, IT, LI, NL, SE**

1. Neue, substituierte 8-Thiotetrahydrochinoline der Formel (I)

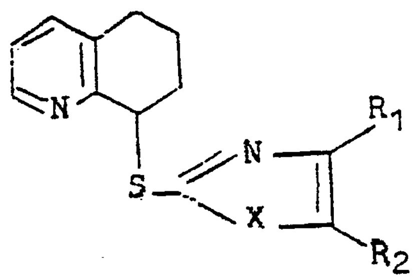

(I)

worin X für ein Sauerstoffatom, ein Schwefelatom, eine Gruppe –NH–, N-alk, worin alk einen Alkylrest mit 1 bis 6 Kohlenstoffatomen bedeutet, oder eine Gruppe

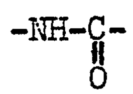

steht, $R_1$ und $R_2$, die gleich oder voneinander verschieden sind, ein Wasserstoffatom, eine Phenylgruppe oder eine Naphthylgruppe bedeuten oder gemeinsam mit den Kohlenstoffatomen, an die sie gebunden sind, eine Phenylgruppe bilden, die gegebenenfalls durch einen Alkylrest mit 1 bis 6 Kohlenstoffatomen, durch einen Alkoxyrest mit 1 bis 6 Kohlenstoffatomen, durch einen Alkylthiorest mit 1 bis 6 Kohlenstoffatomen, durch ein oder mehrere Halogenatome, durch eine $-NO_2$-Gruppe, durch eine Trifluormethylgruppe oder durch eine Gruppe

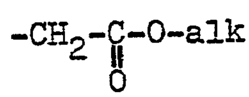

substituiert ist, worin alk für einen Alkylrest mit 1 bis 6 Kohlenstoffatomen steht, mit der Maßgabe, daß X keine Gruppe –NH– bedeuten kann, wenn $R_1$ und $R_2$ gemeinsam mit den Kohlenstoffatomen, an die sie gebunden sind, eine gegebenenfalls substituierte Phenylgruppe bilden, sowie deren Additionssalze mit Mineral- oder organischen Säuren.

2. Neue, substituierte 8-Thiotetrahydrochinoline gemäß Anspruch 1 und deren Salze, dadurch gekennzeichnet, daß in der Formel (I) $R_1$ und $R_2$ gemeinsam mit den Kohlenstoffatomen, an die sie gebunden sind, eine Phenylgruppe bilden, die gegebenenfalls durch einen Alkylrest mit 1 bis 4 Kohlenstoffatomen, durch einen Alkoxyrest mit 1 bis 4 Kohlenstoffatomen, durch einen Alkylthiorest mit 1 bis 4 Kohlenstoffatomen, durch ein oder mehrere Halogenatome, durch eine $-NO_2$-Gruppe, durch eine Trifluormethylgruppe oder durch eine Gruppe

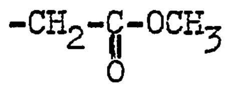

oder

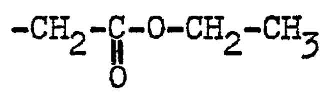

substituiert ist, und X die angegebene Bedeutung besitzt.

3. Neue, substituierte 8-Thiotetrahydrochinoline gemäß Anspruch 1 oder 2 und deren Salze, dadurch gekennzeichnet, daß in der Formel (I) X für ein Schwefelatom oder einen Rest –NH steht und $R_1$ und $R_2$die angegebenen Bedeutungen besitzen.

4. Eines der Derivate der Formel (I) gemäß Anspruch 1 mit den folgenden Bezeichnungen:
8-(2-Benzothiazolylthio)-5,6,7,8-tetrahydrochinolin,
8-[(5-Chlor-2-benzothiazolyl)-thio]-5,6,7,8-tetrahydrochinolin,
8-[(6-Chlor-2-benzothiazolyl)-thio]-5,6,7,8-tetrahydrochinolin,
8-[(6-Ethoxy-2-benzothiazolyl)-thio]-5,6,7,8-tetrahydrochinolin,
8-[(6-Ethylthio-2-benzothiazolyl)-thio]-5,6,7,8-tetrahydrochinolin,
8-[(6-Nitro-2-benzothiazolyl)-thio]-5,6,7,8-tetrahydrochinolin,
8-(4,5-Diphenyl-1H-imidazol-2-yl-thio)-5,6,7,8-tetrahydrochinolin
und deren Salze.

5. Verfahren zur Herstellung der neuen, substituierten 8-Thiotetrahydrochinoline der Formel (I) gemäß Anspruch 1 sowie von deren Salzen, dadurch gekennzeichnet, daß man eine Verbindung der Formel (II)

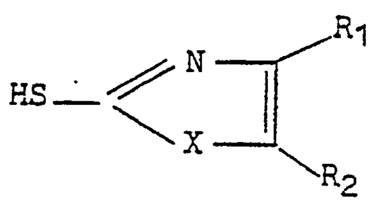

(II)

worin X, $R_1$ und $R_2$ die angegebenen Bedeutungen besitzen, mit einer Verbindung der Formel (III)

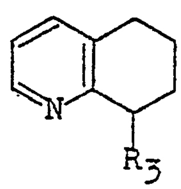

(III)

worin $R_3$ ein Halogenatom bedeutet, umsetzt, um zu der gewünschten Verbindung der Formel (I)

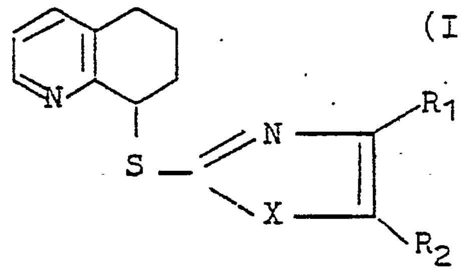

(I)

zu gelangen, worin X, $R_1$ und $R_2$ die angegebenen Bedeutungen besitzen, und gewünschtenfalls das so erhaltene Produkt der Formel (I) in ein Salz überführt.

6. Verfahren gemäß Anspruch 5, dadurch gekennzeichnet, daß das Halogenatom, das von $R_3$ wiedergegeben wird, ein Chlor-, Brom- oder Jodatom ist, die verwendete Verbindung der Formel (II) in Form des Alkalisalzes vorliegt, das vorzugsweise durch Umsetzung eines Alkalimetallhydrids mit der Verbindung der Formel (II) erhalten wird, die Umsetzung der Verbindungen der Formeln (II) und (III) in einem organischen Lösungsmittel stattfindet.

7. Arzneimittel, dadurch gekennzeichnet, daß sie aus den neuen, substituierten 8-Thiotetrahydrochinolinen der Formel (I) gemäß Anspruch 1 sowie aus deren pharmazeutisch verträglichen Salzen bestehen.

8. Arzneimittel, dadurch gekennzeichnet, daß sie aus den neuen, substituierten 8-Thiotetrahydrochinolinen gemäß einem der Ansprüche 2 oder 3 sowie aus deren pharmazeutisch verträglichen Salzen bestehen.

9. Arzneimittel, dadurch gekennzeichnet, daß sie aus den neuen, substituierten 8-Thiotetrahydrochinolinen gemäß Anspruch 4 sowie aus deren pharmazeutisch verträglichen Salzen bestehen.

10. Pharmazeutische Zusammensetzungen, dadurch gekennzeichnet, daß sie als Wirkstoff zumindest eines der Arzneimittel gemäß einem der Ansprüche 7 bis 9 enthalten.

**Patentansprüche für die Vertragsstaaten: AT, BE, LU**

1. Neue, substituierte 8-Thiotetrahydrochinoline der Formel (I)

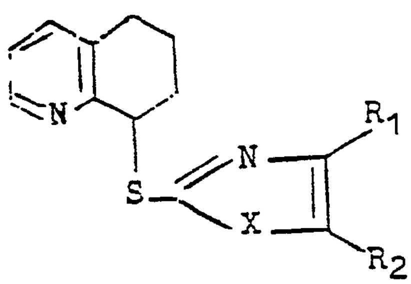

(I)

worin X für ein Sauerstoffatom, ein Schwefelatom, eine Gruppe –NH–, N-alk, worin alk einen Alkylrest mit 1 bis 6 Kohlenstoffatomen bedeutet, oder eine Gruppe

$$-NH-\underset{\underset{O}{\|}}{C}-$$

steht, $R_1$ und $R_2$, die gleich oder voneinander verschieden sind, ein Wasserstoffatom, eine Phenylgruppe oder eine Naphthylgruppe bedeuten oder gemeinsam mit den Kohlenstoffatomen, an die sie gebunden sind, eine Phenylgruppe bilden, die gegebenenfalls durch einen Alkylrest mit 1 bis 6 Kohlenstoffatomen, durch einen Alkoxyrest mit 1 bis 6 Kohlenstoffatomen, durch einen Alkylthiorest mit 1 bis 6 Kohlenstoffatomen, durch ein oder mehrere Halogenatome, durch eine $-NO_2$-Gruppe, durch eine Trifluormethylgruppe oder durch eine Gruppe

$$-CH_2-\underset{\underset{O}{\|}}{C}-O-alk$$

substituiert ist, worin alk für einen Alkylrest mit 1 bis 6 Kohlenstoffatomen steht, sowie deren Additionssalze mit Mineral- oder organischen Säuren.

2. Neue, substituierte 8-Thiotetrahydrochinoline gemäß Anspruch 1 und deren Salze, dadurch gekennzeichnet, daß in der Formel (I) $R_1$ und $R_2$ gemeinsam mit den Kohlenstoffatomen, an die sie gebunden sind, eine Phenylgruppe bilden, die gegebenenfalls durch einen Alkylrest mit 1 bis 4 Kohlenstoffatomen, durch einen Alkoxyrest mit 1 bis 4 Kohlenstoffatomen, durch einen Alkylthiorest mit 1 bis 4 Kohlenstoffatomen, durch ein oder mehrere Halogenatome, durch eine $-NO_2$-Gruppe, durch eine Trifluormethylgruppe oder durch eine Gruppe

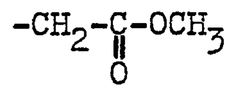

oder

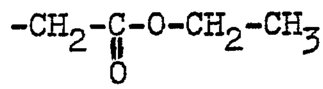

substituiert ist, und X die angegebene Bedeutung besitzt.

3. Neue, substituierte 8-Thiotetrahydrochinoline gemäß Anspruch 1 oder 2 und deren Salze, dadurch gekennzeichnet, daß in der Formel (I) X für ein Schwefelatom oder einen Rest –NH steht und $R_1$ und $R_2$ die angegebenen Bedeutungen besitzen.

4. Eines der Derivate der Formel (I) gemäß Anspruch 1 mit den folgenden Bezeichnungen:
8-(2-Benzothiazolylthio)-5,6,7,8-tetrahydcrochinolin,
8-(6-Chlor-1H-benzimidazol-2-yl-thio)-5,6,7,8-tetrahydrochinolin,
8-[(5-Chlor-2-benzothiazolyl)-thio]-5,6,7,8-tetrahydrochinolin,
8-[(6-Chlor-2-benzothiazolyl)-thio]-5,6,7,8-tetrahydrochinolin,
8-[(6-Ethoxy-2-benzothiazolyl)thio]-5,6,7,8-tetrahydrochinolin,
8-[(6-Ethylthio-2-benzothiazolyl)-thio]-5,6,7,8-tetrahydrochinolin,
8-[(6-Nitro-2-benzothiazolyl)-thio]-5,6,7,8-tetrahydrochinolin,
8-(4,5-Diphenyl-1H-imidazol-2-yl-thio)-5,6,7,8-tetrahydrochinolin
und deren Salze.

5. Verfahren zur Herstellung der neuen, substituierten 8-Thiotetrahydrochinoline der Formel (I) gemäß Anspruch 1 sowie von deren Salzen, dadurch gekennzeichnet, daß man eine Verbindung der Formel (II)

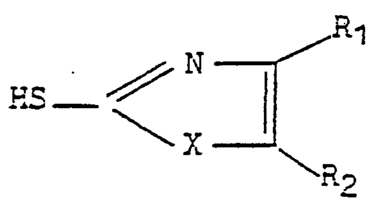

(II)

worin X, $R_1$ und $R_2$ die angegebenen Bedeutungen besitzen, mit einer Verbindung der Formel (III)

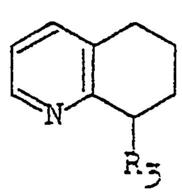

(III)

worin $R_3$ ein Halogenatom bedeutet, umsetzt, um zu der gewünschten Verbindung der Formel (I)

(I)

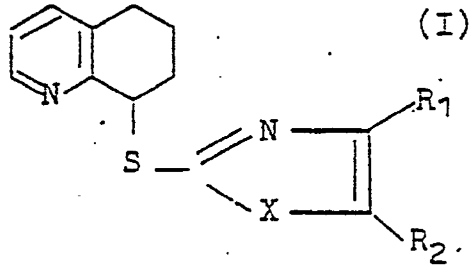

zu gelangen, worin X, $R_1$ und $R_2$ die angegebenen Bedeutungen besitzen, und gewünschtenfalls das so erhaltene Produkt der Formel (I) in ein Salz überführt.

6. Verfahren gemäß Anspruch 5, dadurch gekennzeichnet, daß das Halogenatom, das von $R_3$ wiedergegeben wird, ein Chlor-, Brom- oder Jodatom ist, die verwendete Verbindung der Formel (II) in Form des Alkalisalzes vorliegt, das vorzugsweise durch Umsetzung eines Alkalimetallhydrids mit der Verbindung der Formel (II) erhalten wird, die Umsetzung der Verbindungen der Formeln (II) und (III) in einem organischen Lösungsmittel stattfindet.

7. Arzneimittel, dadurch gekennzeichnet, daß sie aus den neuen, substituierten 8-Thiotetrahydrochinolinen der Formel (I) gemäß Anspruch 1 sowie aus deren pharmazeutisch verträglichen Salzen bestehen.

8. Arzneimittel, dadurch gekennzeichnet, daß sie aus den neuen, substituierten 8-Thiotetrahydrochinolinen gemäß einem der Ansprüche 2 oder 3 sowie aus deren pharmazeutisch verträglichen Salzen bestehen.

9. Arzneimittel, dadurch gekennzeichnet, daß sie aus den neuen, substituierten 8-Thiotetrahydrochinolinen gemäß Anspruch 4 sowie aus deren pharmazeutisch verträglichen Salzen bestehen.

10. Pharmazeutische Zusammensetzungen, dadurch gekennzeichnet, daß sie als Wirkstoff zumindest eines der Arzneimittel gemäß einem der Ansprüche 7 bis 9 enthalten.